(19) Europäisches Patentamt
European Patent Office
Office européen des brevets

(11) Veröffentlichungsnummer: **0 263 066**
**A2**

(12) # EUROPÄISCHE PATENTANMELDUNG

(21) Anmeldenummer: **87810544.4**

(22) Anmeldetag: **21.09.87**

(51) Int. Cl.[4]: **C 07 D 413/04**
C 07 D 417/04, A 01 N 43/82

(30) Priorität: **26.09.86 CH 3870/86**

(43) Veröffentlichungstag der Anmeldung:
**06.04.88 Patentblatt 88/14**

(84) Benannte Vertragsstaaten:
**AT BE CH DE ES FR GB GR IT LI LU NL**

(71) Anmelder: **CIBA-GEIGY AG**
**Klybeckstrasse 141**
**CH-4002 Basel (CH)**

(72) Erfinder: **Beriger, Ernst, Dr.**
**Grabenmattweg 29**
**CH-4123 Allschwil (CH)**

**Eckhardt, Wolfgang, Dr.**
**Breslauerstrasse 14**
**D-7850 Lörrach (DE)**

**Nyfeler, Robert, Dr.**
**Bärenfelserstrasse 8**
**CH-4057 Basel (CH)**

Die Bezeichnung der Erfindung wurde geändert (Richtlinien für die Prüfung im EPA, A-III, 7.3).

(54) **2-Mercapto-oxadiazol- und -thiadiazol-Derivate, Verfahren zu ihrer Herstellung und diese enthaltende nematizide Mittel.**

(57) Neue Verbindungen der Formel

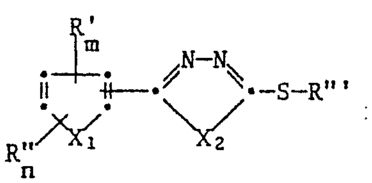

in welcher
R′ $C_1$-$C_5$-Alkyl, Halogen, Trifluormethyl oder $C_1$-$C_3$-Alkoxy;
R″ $C_1$-$C_5$-Alkyl oder Halogen; und
R‴ unsubstituiertes oder durch 1 bis 6 Halogenatome substituiertes $C_1$-$C_5$-Alkyl, unsubstituiertes oder durch 1 bis 6 Halogenatome substituiertes $C_1$-$C_5$-Alkenyl oder unsubstituiertes oder durch 1 bis 6 Halogenatome substituiertes $C_1$-$C_5$-Alkinyl bedeuten;
m für 0, 1, 2 oder 3 und
n für 0 oder 1 stehen; und
$X_1$ und $X_2$ unabhängig voneinander Sauerstoff oder Schwefel darstellen,
als Wirkstoffe zur Bekämpfung von pflanzenparasitären Nematoden und zur Verhütung von Schäden an Kulturpflanzen, die durch Nematodenbefall hervorgerufen werden.

EP 0 263 066 A2

**Beschreibung**

Nematizide Mittel

Die vorliegende Erfindung betrifft neue substituierte 2-Mercapto-5-furyl-1,3,4-oxadiazol- und 2-Mercapto-5-furyl-1,3,4-thiadiazol-Derivate, sowie 2-Mercapto-5-thienyl-1,3,4-oxadiazol und 2-Mercapto-5-thienyl-1,3,4-thiadiazol-Derivate, deren Herstellung sowie nematizide Mittel, die als Wirkstoff mindestens eine dieser Verbindungen enthalten. Die Erfindung betrifft ferner die Verwendung der neuen Wirkstoffe und Mittel zur Bekämpfung von Nematoden, insbesondere von pflanzenschädigenden Nematoden.

Die erfindungsgemässen Verbindungen entsprechen der allgemeinen Formel I

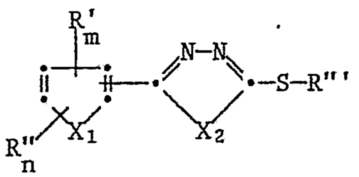 (I),

in welcher
R′ $C_1$-$C_5$-Alkyl, Halogen, Trifluormethyl oder $C_1$-$C_3$-Alkoxy;
R″ $C_1$-$C_5$-Alkyl oder Halogen; und
R‴ unsubstituiertes oder durch 1 bis 6 Halogenatome substituiertes $C_1$-$C_5$-Alkyl, unsubstituiertes oder durch 1 bis 6 Halogenatome substituiertes $C_1$-$C_5$-Alkenyl oder unsubstituiertes oder durch 1 bis 6 Halogenatome substituiertes $C_1$-$C_5$-Alkinyl bedeuten;
m für 0, 1, 2 oder 3 und
n für 0 oder 1 stehen; und
$X_1$ und $X_2$ unabhängig voneinander Sauerstoff oder Schwefel darstellen.

Unter Alkyl sind als selbständiger Rest sowie als Teil einer anderen Gruppe, wie Alkoxy, gerad- und verzweigtkettige Alkylgruppen zu verstehen. Dazu zählen die Methyl-, die Ethyl- sowie die Isomeren der Propyl-, der Butyl- und der Pentylgruppe, darunter insbesondere Methyl, Ethyl, n-Propyl, Isopropyl, Isobutyl oder n-Pentyl. Halogensubstituiertes Alkyl steht für einen einfach bis perhalogenierten Alkylrest, wie z.B. $CHCl_2$, $CH_2F$, $CCl_3$, $CH_2Cl$; $CHFCH_3$, $CH_2CH_2Br$, $CF_2CF_3$, $C_2Cl_5$, $CH_2Br$, $CHBrCl$ usw., vorzugsweise für $CF_3$ und $CHF_2$. Alkenyl bedeutet z.B. Propenyl-(1), Allyl, Butenyl-(1), Butenyl-(2) oder Butenyl-(3) sowie Ketten mit mehreren Doppelbindungen. Alkinyl bedeutet z.B. Propinyl-(2), Butinyl-(1), Butinyl-(2), Pentinyl-(4) usw., vorzugsweise Propargyl. Halogen steht für Fluor, Chlor, Brom oder Jod, vorzugsweise für Fluor, Chlor oder Brom.

Es sind bereits Oxadiazol- und Thiadiazol-Derivate bekannt, die als nematizid wirksam beschrieben sind. So sind in der US-Patentschrift 3,770,754 solche Verbindungen mit einer 1,2,4-Stellung der Heteroatome offenbart, während in der US-Patentschrift 4,454,147 1,3,4-Thiadiazol-Derivate beschrieben sind, bei denen im Vergleich mit den erfindungsgemässen Verbindungen der Heterocyclus anstelle der Mercapto-Gruppen durch ein Chloratom substituiert ist. Diese bekannten Verbindungen haben bisher als Nematizide die in der Praxis an sie gestellten Ansprüche nicht in vollem Umfang befriedigen können. Ferner sind Oxadiazol-Derivate mit fungizider Wirksamkeit in der DE-OS 2 361 613 beschrieben. Es werden darin jedoch keine dieser Verbindungen expressis verbis genannt, die unter dem Umfang der erfindungsgemässen Formel I fallen.

Durch die Bereitstellung der erfindungsgemässen Verbindungen der Formel I ist es nun gelungen einen wertvollen Beitrag zur Bekämpfung der beträchtliche landwirtschaftliche Schäden an Pflanzgut verursachenden Pflanzennematoden zu leisten. Auf diese Weise können Ernteeinbussen bei Kulturpflanzen wie z.B. Kartoffeln, Getreide, Rüben, Raps, Kohl, Tabak, Sojabohnen, Baumwolle und Gemüse sowie Schäden in Baumschulen und im Zierpflanzenbau nachhaltig eingedämmt werden. Die erfindungsgemässen Verbindungen zeichnen sich dabei insbesondere in der wirkungsvollen Bekämpfung von wurzelparasitären Bodennematoden wie z.B. solchen der Gattungen Heterodera und Globodera (zystenbildende Nematoden), Meloidogyne (Wurzelgallennematoden) sowie der Gattungen Radopholus, Pratylenchus, Tylenchulus, Longidorus, Trichodorus und Xiphinema aus. Ferner lassen sich mit den erfindungsgemässen Wirkstoffen die Nematodengattungen Ditylenchus (Stengelparasiten) Aphelenchoides (Blattnematoden) und Anguina (Blütennematoden) wirkungsvoll bekämpfen.

Mit den Wirkstoffen der Formel I lassen sich vorzugsweise besonders schädliche Nematodenarten der Gattung Meloidogyne, wie z.B. Meloidogyne incognita, sowie der Gattung Heterodera, wie z.B. Heterodera glycines (Sojabohnen-Zystennematode) und ferner der Gattung Globodera, wie z.B. Globodera rostochiensis (Kartoffelzystennematode) sowie Vertreter von wandernden Endoparasiten, wie z.B. Pratylenchus penetrans oder Radopholus similis und Vertreter von Ektoparasiten, wie z.B. Trichodorus spp. und Xiphinema spp. erfolgreich bekämpfen.

Zur Bekämpfung der Pflanzennematoden und zur Gesunderhaltung des Pflanzgutes können die neuen Wirkstoffe kurativ, präventiv oder systemisch eingesetzt werden. Dabei entfalten sie eine breit gefächerte Aktivität gegen die verschiedenen Nematodenspecies und werden somit den Erfordernissen der Praxis gerecht. Die nematizide Wirkungsweise der erfindungsgemässen Verbindungen wird durch eine geringe

Phytotoxizität in vorteilhafter Weise begleitet, womit der allgemein wünschenswerten Verminderung der Umweltbelastung in besonderem Masse Rechnung getragen wird.

Im Rahmen der vorliegenden Erfindung sind folgende Verbindungsgruppen der Formel I bevorzugt:

1) Verbindungen, in denen
R′ $C_1$-$C_5$-Alkyl, Halogen, Trifluormethyl oder $C_1$-$C_3$-Alkoxy;
R″ $C_1$-$C_5$-Alkyl oder Halogen; und
R‴unsubstituiertes oder durch 1 bis 6 Halogenatome substituiertes $C_1$-$C_5$-Alkyl, unsubstituiertes oder durch 1 bis 6 Halogenatome substituiertes $C_1$-$C_5$-Alkenyl oder unsubstituiertes oder durch 1 bis 6 Halogenatome substituiertes $C_1$-$C_5$-Alkinyl bedeuten;
m für 0, 1, 2 oder 3 und
n für 0 oder 1 stehen; und
$X_1$ und $X_2$ unabhängig voneinander Sauerstoff oder Schwefel darstellen, mit der Massgabe, dass wenn m und n zusammen Null sind oder wenn $X_1$ Schwefel und R′ und R″ zusammen 3 Chloratome darstellen, R‴ nicht Alkenyl oder Alkinyl sein darf.

2) Verbindungen, in denen
R′ $C_1$-$C_2$-Alkyl, Halogen, Trifluormethyl oder $C_1$-$C_2$-Alkoxy;
R″ $C_1$-$C_2$-Alkyl oder Halogen;
R‴ unsubstituiertes oder durch 1 bis 3 Halogenatome substituiertes $C_1$-$C_3$-Alkyl, unsubstituiertes oder durch 1 bis 3 Halogenatome substituiertes $C_1$-$C_3$-Alkenyl oder unsubstituiertes oder durch 1 bis 3 Halogenatome substituiertes $C_1$-$C_3$-Alkinyl bedeuten;
m für 0, 1 oder 2 und
n für 0 oder 1 stehen; und
$X_1$ und $X_2$ unabhängig voneinander Sauerstoff oder Schwefel darstellen.

3) Verbindungen, in denen
R′ $C_1$-$C_2$-Alkyl, Halogen, Trifluormethyl oder $C_1$-$C_2$-Alkoxy;
R″ $C_1$-$C_2$-Alkyl oder Halogen;
R‴ unsubstituiertes oder durch 1 bis 3 Halogenatome substituiertes $C_1$-$C_3$-Alkyl, unsubstituiertes oder durch 1 bis 3 Halogenatome substituiertes $C_1$-$C_3$-Alkenyl oder unsubstituiertes oder durch 1 bis 3 Halogenatome substituiertes $C_1$-$C_3$-Alkinyl bedeuten;
m für 0, 1 oder 2 und
n für 0 oder 1 stehen; und
$X_1$ und $X_2$ unabhängig voneinander Sauerstoff oder Schwefel darstellen, mit der Massgabe, dass wenn m und n zusammen Null sind oder wenn $X_1$ Schwefel und R′ und R″ zusammen 3 Chloratome darstellen, R‴ nicht Alkenyl oder Alkinyl sein darf.

4) Verbindungen, in denen
R′ Methyl, Chlor, Brom oder Methoxy;
R″ Methyl, Chlor Brom oder Jod;
R‴ unsubstituiertes oder durch 1 bis 3 Fluoratome substituiertes $C_1$-$C_3$-Alkyl, unsubstituiertes oder durch 1 bis 3 Fluoratome substituiertes $C_1$-$C_3$-Alkenyl oder unsubstituiertes oder durch 1 bis 3 Fluoratome substituiertes $C_1$-$C_3$-Alkinyl bedeuten;
m und n unabhängig für 0 oder 1 stehen; und
$X_1$ und $X_2$ unabhängig voneinander Sauerstoff oder Schwefel darstellen.

5) Verbindungen, in denen
R′ Methyl, Chlor, Brom oder Methoxy;
R″ Methyl, Chlor Brom oder Jod;
R‴ unsubstituiertes oder durch 1 bis 3 Fluoratome substituiertes $C_1$-$C_3$-Alkyl, unsubstituiertes oder durch 1 bis 3 Fluoratome substituiertes $C_1$-$C_3$-Alkenyl oder unsubstituiertes oder durch 1 bis 3 Fluoratome substituiertes $C_1$-$C_3$-Alkinyl bedeuten;
m und n unabhängig für 0 oder 1 stehen; und
$X_1$ und $X_2$ unabhängig voneinander Sauerstoff oder Schwefel darstellen, mit der Massgabe, dass wenn m und n zusammen Null sind oder wenn $X_1$ Schwefel und R′ und R″ zusammen 3 Chloratome darstellen, R‴ nicht Alkenyl oder Alkinyl sein darf.

6) Verbindungen, in denen
R′ Methyl, Chlor, Brom oder Methoxy;
R″ Methyl, Chlor oder Brom; und
R‴ unsubstituiertes oder durch 1 bis 3 Fluoratome substituiertes $C_1$-$C_3$-Alkyl bedeuten;
m und n unabhängig voneinander für 0 oder 1 stehen; und
$X_1$ und $X_2$ unabhängig voneinander Sauerstoff oder Schwefel darstellen.

7) Verbindungen, in denen
R′ Methyl, Chlor, Brom oder Methoxy;
R″ Methyl, Chlor oder Brom; und
R‴ $CHF_2$ bedeuten; und
m und n unabhängig voneinander für 0 oder 1 stehen; und
$X_1$ und $X_2$ unabhängig voneinander Sauerstoff oder Schwefel darstellen.

Bevorzugte Verbindungen sind;

2-Difluormethylthio-5-thien-2-yl-1,3,4-thiadiazol,
2-Difluormethylthio-5-(5-chlorthien-2-yl)-1,3,4-thiadiazol,
2-Difluormethylthio-5-(5-methylthien-2-yl)-1,3,4-thiadiazol,
2-Difluormethylthio-5-(5-bromthien-2-yl)-1,3,4-thiadiazol,
2-Difluormethylthio-5-thien-2-yl-1,3,4-oxadiazol,
2-Difluormethylthio-5-(chlorthien-2-yl)-1,3,4-oxadiazol,
2-Difluormethylthio-5-(5-methylthien-2-yl)-1,3,4-oxadiazol,
2-Difluormethylthio-5-(furan-2-yl)-1,3,4-thiadiazol,
2-Difluormethylthio-5-(2-methylfuran-3-yl)-1,3,4-thiadiazol,
2-Difluormethylthio-5-(4-methylthien-2-yl)-1,3,4-thiadiazol,
2-Difluormethylthio-5-(furan-2-yl)-1,3,4-oxadiazol

Verbindungen der Formel I werden erfindungsgemäss hergestellt, indem man

a) in einer Kondensationsreaktion eine Verbindung der Formel IIa

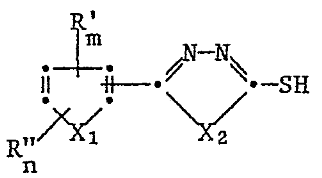

(IIa)

oder eine Verbindung der Formel IIb

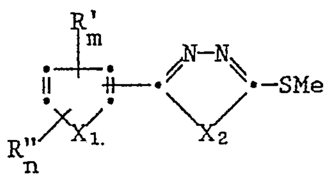

(IIb)

mit einer Verbindung der Formel IIIa

Hal-R‴ (IIIa)

in einem inerten Lösungsmittel oder Lösungsmittel-Gemisch bei erhöhter Temperatur gegebenenfalls in Gegenwart eines Katalysators und gegebenenfalls bei erhöhtem Druck umsetzt, wobei die Umsetzung einer Verbindung der Formel IIa in Gegenwart einer Base verläuft, oder indem man

b) in einer Anlagerungsreaktion eine Verbindung der Formel IIa

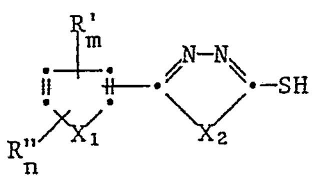

(IIa)

mit einer Verbindung der Formel IIIb

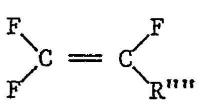

(IIIb)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ia

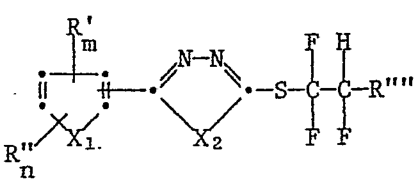

(Ia)

oder zu einer Verbindung der Formel Ib

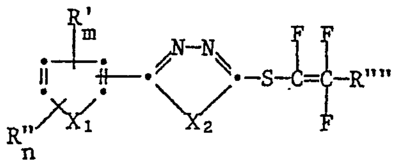 (Ib)

führt.

In den vorstehend genannten Formeln IIa, IIb, Ia, Ib, IIIa und IIIb steht Me für ein Alkalimetall oder für Ammonium, Hal für Halogen, vorzugsweise für Chlor, Brom oder Jod, und R'''' für Fluor oder Trifluormethyl während R', R'', R''', m, $X_1$ und $X_2$ die unter Formel I angegebenen Bedeutungen besitzen.

Für die Herstellung der erfindungsgemässen Wirksubstanzen geeignete Lösungs- oder Verdünnungsmittel sind z.B. Ether und etherartige Verbindungen wie Dialkylether (Diethylether, Diisopropylether, tert.-Butylmethylether usw.), Anisol, Dioxan, Tetrahydrofuran; aliphatische und aromatische Kohlenwasserstoffe wie Benzol, Toluol, Petrolether; halogenierte Kohlenwasserstoffe wie Chlorbenzol, Methylenchlorid, Chloroform, Ethylenchlorid, Tetrachlorkohlenstoff, Tetrachlorethylen; Nitrile wie Acetonitril, Propionitril; N,N-dialkylierte Amide wie Dimethylformamid; Dimethylsulfoxid; Ketone wie Aceton, Diethylketon, Methylethylketon sowie Wasser und Alkohole wie z.B. Methanol, Ethanol, Isopropanol oder Butanol; und ganz allgemein Gemische solcher Lösungsmittel untereinander.

Als Basen kommen organische und anorganische Basen in Betracht; z.B. vorzugsweise tertiäre Amine wie Trialkylamine (Trimethylamin, Triethylamin, Tripropylamin usw.), sowie Oxide, Hydroxide, Carbonate und Hydrogencarbonate von Alkyli- und Erdalkalimetallen (z.B. CaO, BaO, NaOH, KOH, $Ca(OH)_2$, $KHCO_3$, $NaHCO_3$, $Ca(HCO_3)_2$, $K_2CO_3$, $Na_2CO_3$ usw.), ferner Acetate wie z.B. $CH_3COONa$ oder $CH_3COOK$. Darüber hinaus eignen sich als Basen auch Alkalialkoholate wie z.B. Natriumethylat, Natriumpropylat, Kalium-tert.-butylat oder Natriumethylat.

Die Zugabe katalytischer Mengen eines Kronenethers, wie z.B. 18-Krone-6 oder 15-Krone-5, wirkt sich in den Herstellungsverfahren günstig auf den Reaktionsablauf aus. Ferner hat sich für den gleichen Zweck die katalytische Verwendung von Tetraalkylaminsalzen, z.B. Tetraalkylammoniumhydrochloride oder -bromide, vorzugsweise Tetra-n-butylammoniumhydrobromide, als vorteilhaft erwiesen. Darüber hinaus sind Alkalijodide, vorzugsweise Kaliumjodid, als Katalysatoren vorteilhaft einsetzbar.

In den Herstellungsverfahren betragen die Reaktionstemperaturen 10 bis 90° C, vorzugsweise 30° bis 80° C. Für die Druckverhältnisse während des Reaktionsablaufs sind 1 bis 20 bar, vorzugsweise 6 bis 14 bar, massgebend.

Die Erfindung betrifft auch Mittel, zur Bekämpfung von pflanzenschädigenden Nematoden sowie zur präventiven Verhütung des Nematodenbefalls von Pflanzgut, welche die Wirkstoffe der Formel I enthalten.

Darüber hinaus schliesst die vorliegende Erfindung zusätzliche die Herstellung nematizider Mittel ein, die gekennzeichnet ist durch das innige Vermischen von Aktivsubstanzen der Formel I mit einer oder mehreren hierin beschriebenen Trägerstoffen und Hilfsingredienzen. Eingeschlossen ist auch ein Verfahren zur Behandlung von Pflanzen, das durch die Applikation der Verbindungen der Formel I bzw. der neuen Mittel charakterisiert ist.

Die Ausgangsverbindungen der Formeln IIa und IIb sind teils bekannt, teils neu. Die neuen Verbindungen der genannten Formeln sind Zwischenprodukte zur Herstellung wertvoller nematozider Wirkstoffe (siehe Tabelle 0) und bilden damit einen Bestandteil der vorliegenden Erfindung.

Die Ausgangsverbindungen der Formeln IIa und IIb können nach bekannten Methoden wie folgt hergestellt werden:

a) Die 2-Mercapto-1,3,4-oxadiazole sind gewinnbar durch Zugabe von Schwefelkohlenstoff zu einer Lösung des entsprechend substituierten Furoyl- oder Thenoylhydrazids in alkoholisch-wässrigem Kaliumhydroxid und Erhitzen des Reaktionsgemisches während einiger Stunden. Als Lösungsmittel werden dabei Alkohole wie z.B. Ethylalkohol oder n-Amylalkohol verwendet. Durch Ansäuern der gebildeten Kaliumsalze werden die freien Mercapto-Verbindungen erhalten [vgl. J. Am. Chem. Soc. 78, 4975-4978 (1956)].

b) Die 2-Mercapto-1,3,4-thiadiazole sind erhältlich durch Behandlung des entsprechend substituierten Furoyl- oder Thenoyl-kaliumdithiocarbazats mit konzentrierter Schwefelsäure bei -5° bis 10° C [vgl. J. prakt. Chem. 93, 49 (1916); J. Org. Chem. 23, 1021 (1958); J. Heterocycl. Chem. 19, 542-544 (1982)].

Die Ausgangsverbindungen der Formeln IIa und IIb sind z.T. neue Stoffe, die als Zwischenprodukte zur Herstellung wertvoller, nützlicher Endprodukte der Formel I einen weiteren Aspekt der vorliegenden Erfindung darstellen (z.B. die Verbindungen der Tabelle 0).

Ein bevorzugtes Verfahren für den Einsatz eines Wirkstoffs der Formel I bzw. eines nematiziden Mittels, das mindestens einen dieser Wirkstoffe enthält, ist das Einbringen in den Erdboden. Dabei wird der Standort der Pflanzen mit einer flüssigen oder festen Zubereitung behandelt.

Die Verbindungen der Formel I können aber auch auf Samenkörner aufgebracht werden (Beizung/Coating), indem man die Körner entweder in einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel, Knospen oder Blätter.

Wirkstoffe der Formel I werden üblicherweise in Form von formulierten Zusammensetzungen verwendet und können gleichzeitig oder nacheinander mit weiteren Wirkstoffen auf die zu behandelnde Fläche oder Pflanze gegeben werden. Diese weiteren Wirkstoffe können auch andere in der Landwirtschaft angewendete

Mittel umfassen, die in ihrer Nutzanwendung der Produktionssteigerung durch Förderung des Nutzpflanzenwachstums dienen, wie Düngemittel, Herbizide, Insektizide, Fungizide, Molluskizide u.a., oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel I werden in unveränderter Form oder vorzugsweise zusammen mit den in der Formulierungstechnik üblichen Hilfsmitteln eingesetzt. Sie werden z.B. zu Emulsionskonzentraten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Verstäuben, Verstreuen oder Giessen werden gleich wie die Art der Mittel den angestrebten Zielen und den gegebenen Verhältnissen entsprechend gewählt. Günstige Aufwandmengen liegen im allgemeinen bei 500 g bis 6 kg Aktivsubstanz (AS) je ha; bevorzugt 1 bis 4 kg AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenfalls mit oberflächenaktiven Substanzen (Tensiden).

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Substanzen kommen je nach Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sogenannte synthetische Tenside verwendet, insbesondere Fettsulfonate, Fettsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. Das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken.

1. Herstellungsbeispiele

Beispiel 1.1: Herstellung von

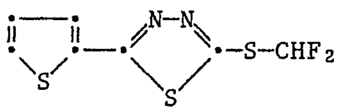

2-Difluormethylthio-5-thienyl-1,3,4-thiadiazol

Zu einer Lösung von 2,9 g Kaliumhydroxid in 10 ml Wasser gibt man unter Rühren 6,0 g 2-Mercapto-5-thienyl-1,3,4-thiadiazol und 45 ml Dioxan. Nach Zugabe von 0,2 g Kaliumjodid und 0,1 g Tetrabutylammoniumbromid erwärmt man die Mischung auf 40° C und leitet unter kräftigem Rühren während 3 Stunden einen schwachen Strom von Chlordifluormethan ein. Anschliessend dampft man die Reaktionsmischung im Vakuum ein. Der Eindampfrückstand wird in Methylenchlorid aufgenommen und nacheinander mit Wasser und 1-normaler Natronlauge ausgewaschen. Nach dem Abdampfen des Lösungsmittels erhält man die Titelverbindung (aus Cyclohexan umkristallisiert) als farblose Kristalle vom Schmelzpunkt 63-64° C.

Beispiel 1.2: Herstellung von

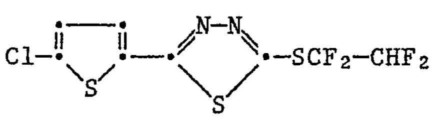

2-(1,1,2,2-Tetrafluorethylthio)-5-(5'-chlorthien-2'-yl)-1,3,4-thiadiazol

Zu einer Lösung von 7,1 g 5-(5'-Chlorthien-2'-yl)-2-mercapto-1,3,4-thiadiazol in 60 ml Dimethylformamid gibt man 0,66 g Kaliumhydroxid und verrührt die Mischung in einem Druckgefäss. Man presst 16 g Tetrafluorethylen hinein und erwärmt die Reaktionsmischung während 20 Stunden auf 60° C (10-12•$10^5$ Pa). Nach Abkühlung und Entspannung des Ueberdruckes giesst man die Lösung auf 400 ml Wasser und extrahiert das Reaktionsprodukt mit Toluol. Nach dem Abdestillieren des Toluols erhält man als Rückstand 8,7 g Rohprodukt der Titelverbindung, das nach dem Umkristallisieren aus Hexan den Schmelzpunkt 66-68° C hat (Ausbeute: 6,8 g).

Beispiel 1.3: Herstellung von

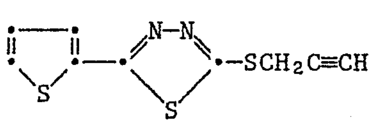

2-Propargylthio-5-thien-2-yl-1,3,4-thiadiazol

6 g 2-Mercapto-5-thienyl-1,3,4-thiadiazol werden in 40 ml Dioxan vorgelegt und während 15 Minuten mit 3,36 g Kalium-t-butylat verrührt. Anschliessend tropft man bei 30°C 5,9 g Propargylbromid zu und rührt die Reaktionsmischung über Nacht bei Raumtemperatur. Man filtriert die Salze ab, dampft das Filtrat ein, nimmt den Rückstand in Methylenchlorid auf und wäscht die Lösung mit Wasser, dann mit 1-normaler Natronlauge aus. Man entfernt das Lösungsmittel im Vakuum und erhält als Rückstand 6,1 g Rohprodukt der Titelverbindung, das aus t-Butylmethyläther umkristallisiert die Titelverbindung vom Schmelzpunkt 71-73°C ergibt.

Analog der vorstehenden Herstellungsbeispiele sowie der vorbeschriebenen Verfahren lassen sich folgende erfindungsgemässe Verbindungen herstellen. Die nachfolgend aufgeführten Verbindungen dienen der Illustration der vorliegenden Erfindung und stellen keine Begrenzung derselben dar.

Tabelle 0

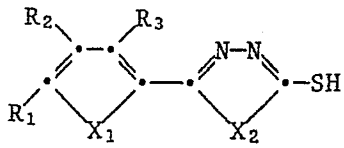

| Verb-Nr. | $R_1$ | $R_2$ | $R_3$ | $X_1$ | $X_2$ | Physikal. Daten |
|---|---|---|---|---|---|---|
| 0.1 | H | H | H | S | S | Smp. 186-190°C |
| 0.2 | H | H | H | S | O | Smp. 197-199°C |
| 0.3 | H | H | H | O | S | Smp. 225-227°C |
| 0.4 | Cl | H | H | S | S | Smp. 222-224°C |
| 0.5 | Br | H | H | S | S | Smp. 220°C |
| 0.6 | Br | Br | H | S | S | Smp. 218-221°C |
| 0.7 | $CH_3$ | H | H | S | S | Smp. 236-239°C |
| 0.8 | H | $CH_3$ | H | S | S | Smp. 213-216°C |
| 0.9 | H | H | $CH_3$ | S | S | Smp. 200-202°C |
| 0.10 | Cl | Cl | H | S | S | |
| 0.11 | Cl | Cl | Cl | S | O | |
| 0.12 | Cl | H | H | S | O | Smp. 202-204°C |
| 0.13 | $CH_3$ | H | H | S | O | Smp. 204-207°C |
| 0.14 | H | $CH_3$ | H | S | O | |
| 0.15 | H | H | H | O | O | Smp. 171-173°C |
| 0.16 | Br | H | H | O | O | Smp. 175-177°C |
| 0.17 | $CH_3$ | H | H | O | O | Smp. 163-166°C |
| 0.18 | $CH_3$ | H | H | O | S | Smp. 190-194°C |
| 0.19 | Br | H | H | O | S | Smp. ab 160°C Zers. |
| 0.20 | Br | Br | H | S | O | Smp. 217-219°C |
| 0.21 | Cl | H | H | O | O | Smp. 150-155°C |
| 0.22 | Br | H | H | S | O | Smp. 202-204°C |

Tabelle 1

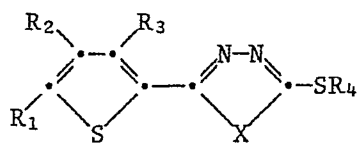

| Verb-Nr. | $R_1$ | $R_2$ | $R_3$ | X | $R_4$ | Physikal. Daten |
|---|---|---|---|---|---|---|
| 1.1 | H | H | H | S | $CHF_2$ | Smp. 63-64°C |
| 1.2 | Cl | H | H | S | $CHF_2$ | Smp. 91-92°C |
| 1.3 | Br | H | H | S | $CHF_2$ | Smp. 92-94°C |
| 1.4 | $CH_3$ | H | H | S | $CHF_2$ | Smp. 93-95°C |
| 1.5 | Cl | Cl | H | S | $CHF_2$ | |
| 1.6 | Cl | Cl | Cl | S | $CHF_2$ | |
| 1.7 | Br | Br | H | S | $CHF_2$ | Smp. 100-110°C |
| 1.8 | H | H | $CH_3$ | S | $CHF_2$ | Smp. 92-94°C |
| 1.9 | H | $CH_3$ | H | S | $CHF_2$ | Smp. 44-46°C |
| 1.10 | H | H | H | S | $CHF_2$ | Smp. 56-58°C |
| 1.11 | Cl | H | H | O | $CHF_2$ | Smp. 73-75°C |
| 1.12 | Br | H | H | O | $CHF_2$ | |
| 1.13 | H | $CH_3$ | H | O | $CHF_2$ | |
| 1.14 | Cl | H | H | O | $CHF_2$ | |
| 1.15 | Cl | H | H | S | $-CF=CFCF_3$ | Smp. 66-68°C |
| 1.16 | J | H | H | S | $-CF_2CHF_2$ | |
| 1.17 | $C_4H_9(t)$ | H | H | S | $-CHF_2$ | |
| 1.18 | H | H | H | S | $-CH_2C{\equiv}CH$ | Smp. 71-73°C |

Tabelle 1 (Fortsetzung)

| Verb-Nr. | $R_1$ | $R_2$ | $R_3$ | X | $R_4$ | Physikal. Daten |
|---|---|---|---|---|---|---|
| 1.19 | Cl | H | H | S | $CH_2C{\equiv}CH$ | Smp. 80-83°C |
| 1.20 | Br | Br | H | S | $CH_2C{\equiv}CH$ | |
| 1.21 | H | H | $CH_3$ | S | $CH_2C{\equiv}CH$ | Smp. 82-84°C |
| 1.22 | $CH_3$ | H | H | S | $CH_2C{\equiv}CH$ | |
| 1.23 | Cl | H | H | O | $CH_2C{\equiv}CH$ | Smp. 106-108°C |
| 1.24 | H | H | H | O | $C_3H_7(i)$ | $n_D^{24}$ 1,5990 |
| 1.25 | Cl | H | H | S | $C_3H_7(i)$ | Smp. 87-88°C |
| 1.26 | Br | H | H | S | $C_3H_7(i)$ | Smp. 72-73°C |
| 1.27 | H | H | $CH_3$ | S | $C_3H_7(i)$ | $n_D^{24}$ 1,6533 |
| 1.28 | Cl | H | H | O | $C_3H_7(i)$ | Smp. 69-70°C |
| 1.29 | Br | Br | H | S | $C_3H_7(i)$ | |
| 1.30 | $CH_3O$ | H | H | S | $C_3H_7(i)$ | |
| 1.31 | Cl | Cl | Cl | S | $C_3H_7(i)$ | |
| 1.32 | H | H | H | S | $CH_2CH{=}CH_2$ | |
| 1.33 | Cl | H | H | S | $CH_2CH{=}CH_2$ | Smp. 78-80°C |
| 1.34 | Br | H | H | S | $CH_2CH{=}CH_2$ | |
| 1.35 | Br | Br | H | S | $CH_2CH{=}CH_2$ | |
| 1.36 | Cl | H | H | O | $CH_2CH{=}CH_2$ | Smp. 54-56°C |
| 1.37 | $CH_3$ | H | H | S | $CH_2CH{=}CH_2$ | |
| 1.38 | $CH_3$ | H | $OCH_3$ | S | $CHF_2$ | Smp. 83-86°C |

<u>Tabelle 1</u> (Fortsetzung)

| Verb-Nr. | $R_1$ | $R_2$ | $R_3$ | X | $R_4$ | Physikal. Daten |
|---|---|---|---|---|---|---|
| 1.39 | H | $CH_3$ | H | S | $CH_2C{\equiv}CH$ | Smp. 96-98°C |
| 1.40 | H | $CH_3$ | H | S | $CH_3H_7(i)$ | Smp. 66-68°C |
| 1.41 | Br | Br | H | O | $CHF_2$ | Smp. 71-73°C |
| 1.42 | F | H | H | S | $CHF_2$ | |
| 1.43 | F | H | H | O | $CHF_2$ | |
| 1.44 | $CH_3$ | H | H | O | $CHF_2$ | Smp. 65-66,5°C |
| 1.45 | $CH_3$ | H | H | O | $CH_2CH_2{=}CH_2$ | Oel |
| 1.46 | $CH_3$ | H | H | O | $C_3H_7(i)$ | Oel |
| 1.47 | $CH_3$ | H | H | O | $CH_2C{\equiv}CH$ | Smp. 68,5-70°C |
| 1.48 | Br | H | H | O | $CH_2C{\equiv}CH$ | Smp. 93-95°C |
| 1.49 | Br | H | H | O | $C_3H_7(i)$ | Smp. 66-68°C |
| 1.50 | Br | H | H | O | $CH_2CH{=}CH_2$ | |
| 1.51 | H | H | H | S | $C_3H_7(i)$ | $n_D^{20}$ 1,6604 |
| 1.52 | Cl | H | H | O | $CF_2CHFCF_3$ | halbfest |
| 1.53 | Cl | H | H | O | $C_3H_7(i)$ | Smp. 69-71,5°C |
| 1.54 | Cl | H | H | O | $CH_2C{\equiv}CH$ | Smp. 106-108°C |
| 1.55 | Cl | H | H | O | $CH_2CH{=}CH_2$ | Smp. 54-56°C |
| 1.56 | Cl | H | H | S | $CF_2CHFCF_3$ | fest |

Tabelle 2

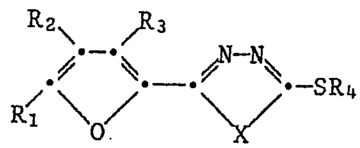

| Verb-Nr. | $R_1$ | $R_2$ | $R_3$ | X | $R_4$ | Physikal. Daten |
|---|---|---|---|---|---|---|
| 2.1 | H | H | H | S | $CHF_2$ | Smp. 66-68°C |
| 2.2 | H | H | H | O | $CHF_2$ | Smp. 65-67°C |
| 2.3 | $CH_3$ | H | H | S | $CHF_2$ | Smp. 49-50°C |
| 2.4 | $CH_3$ | H | H | S | $CH_2CH{=}CH_2$ | $n_D^{23}$ 1,6384 |
| 2.5 | $CH_3$ | H | H | O | $CH_2CH{=}CH_2$ | - |
| 2.6 | $CH_3$ | H | H | S | $CH_2C{\equiv}CH$ | Smp. 73-75,5°C |
| 2.7 | $CH_3$ | H | H | O | $CH_2C{\equiv}CH$ | Smp. 75-77°C |
| 2.8 | H | H | H | S | $CH_2C{\equiv}CH$ | Smp. 82-85°C |
| 2.9 | H | H | H | S | $CH_2CH{=}CH_2$ | Smp. 57-59°C |
| 2.10 | H | H | H | S | $C_3H_7(i)$ | Oel |
| 2.11 | Br | H | H | S | $CHF_2$ | Smp. 84-86°C |
| 2.12 | Br | H | H | O | $CHF_2$ | Smp. 53-56°C |
| 2.13 | H | H | H | S | $CF_2CHF_2$ | |
| 2.14 | H | H | H | S | $CF{=}CFCF_3$ | |
| 2.15 | H | H | H | O | $CH_2CH{=}CH_2$ | Oel |
| 2.16 | $CH_3$ | H | H | S | $-C_3H_7(i)$ | Oel |
| 2.17 | $CH_3$ | H | H | O | $-CHF_2$ | Oel |
| 2.18 | Cl | H | H | S | $-CHF_2$ | Smp. 76-78°C |
| 2.19 | $CH_3$ | H | H | O | $-CHF_2$ | Oel |
| 2.20 | Cl | H | H | S | $-CH_2CH{=}CH_2$ | Smp. 46-48°C |
| 2.21 | Cl | H | H | S | $-C_3H_7(i)$ | Oel |
| 2.22 | Cl | H | H | S | $-CH_2C{\equiv}CH$ | Smp. 70-72°C |
| 2.23 | H | H | H | O | $-C_3H_7(i)$ | Oel |
| 2.24 | Cl | H | H | O | $-CH_2C{\equiv}CH$ | Smp. 98-100°C |
| 2.25 | Cl | H | H | O | $-CHF_2$ | Smp. 50-52°C |
| 2.26 | Cl | H | H | O | $-C_3H_7(i)$ | Smp. 57-59°C |

Tabelle 3

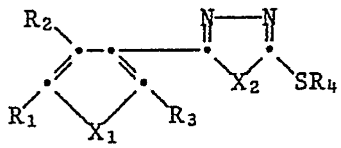

| Verb-Nr. | $R_1$ | $R_2$ | $R_3$ | $X_1$ | $X_2$ | $R_4$ | Physikal. Daten |
|---|---|---|---|---|---|---|---|
| 3.1 | H | H | H | S | S | $CHF_2$ | Smp. 53-55° |
| 3.2 | H | H | H | O | O | $CHF_2$ | |
| 3.3 | H | H | H | S | O | $CHF_2$ | Smp. 83-84° |
| 3.4 | H | H | H | O | S | $CHF_2$ | |
| 3.5 | H | H | $CH_3$ | S | S | $CHF_2$ | |
| 3.6 | H | H | $CH_3$ | O | S | $CHF_2$ | $n_D^{50}$ 1,6075 |
| 3.7 | $CH_3$ | H | $OCH_3$ | S | S | $CHF_2$ | |
| 3.8 | H | H | $CH_3$ | S | S | $CHF_2$ | |
| 3.9 | H | H | $CH_3$ | S | S | $C_3H_7(i)$ | |
| 3.10 | H | H | $CH_3$ | S | S | $CH_2C{\equiv}CH$ | |
| 3.11 | H | H | $CH_3$ | S | S | $CH_2CH{=}CH2$ | |
| 3.12 | H | H | $CH_3$ | S | S | $CF_2CHF_2$ | |
| 3.13 | H | H | $CH_3$ | S | S | $CF{=}CFCF_3$ | |
| 3.14 | Cl | H | $CH_3$ | S | S | $CHF_2$ | |
| 3.15 | Br | H | $CH_3$ | S | S | $CHF_2$ | |
| 3.16 | Cl | H | H | S | S | $CHF_2$ | |
| 3.17 | $CH_3$ | H | $CH_3$ | S | S | $CHF_2$ | |
| 3.18 | Cl | H | $CH_3$ | O | S | $CHF_2$ | |
| 3.19 | Cl | H | $CH_3$ | O | O | $CHF_2$ | |
| 3.20 | $CH_3$ | H | $CH_3$ | O | O | $CHF_2$ | |
| 3.21 | $CH_3$ | H | $CH_3$ | O | S | $CHF_2$ | Smp. 110-113°C |
| 3.22 | $CH_3$ | H | $CH_3$ | S | O | $CHF_2$ | |
| 3.23 | H | H | $CH_3$ | O | S | $CH_2C{\equiv}CH$ | $n_D^{50}$ 1,6359 |
| 3.24 | H | H | $CH_3$ | O | S | $CH_2CH{=}CH_2$ | $n_D^{50}$ 1,6320 |

Tabelle 3 (Fortsetzung)

| Verb-Nr. | $R_1$ | $R_2$ | $R_3$ | $X_1$ | $X_2$ | $R_4$ | Physikal. Daten |
|---|---|---|---|---|---|---|---|
| 3.25 | Cl | H | Cl | S | O | $CHF_2$ | $n_D^{40}$ 1,5928 |
| 3.26 | Cl | H | Cl | S | S | $CHF_2$ | $n_D^{40}$ 1,6438 |
| 3.27 | Cl | H | Cl | S | O | H | Smp. 225-228°C |
| 3.28 | H | H | H | O | S | H | Smp. ab 110°C Zers. |
| 3.29 | H | H | H | O | O | H | Smp. 168-171°C |
| 3.30 | H | H | H | O | O | $CHF_2$ | Oel |
| 3.31 | H | H | H | O | S | $CHF_2$ | Smp. 47-49 |
| 3.32 | Br | H | H | O | S | H | |
| 3.33 | Br | H | H | O | S | $CHF_2$ | |
| 3.34 | Br | H | H | O | O | H | |
| 3.35 | Br | H | H | O | O | $CHF_2$ | |
| 3.36 | H | H | Br | O | S | H | |
| 3.37 | H | H | Br | O | S | $CHF_2$ | |
| 3.38 | H | H | Br | O | O | H | |
| 3.39 | H | H | Br | O | O | $CHF_2$ | |
| 3.40 | H | H | H | S | O | $CH_2CH_2CF{=}CF_2$ | Oel |
| 3.41 | Br | H | H | O | O | $CHF_2$ | Oel |
| 3.42 | Br | H | H | O | S | $CHF_2$ | Oel |

2. Formulierungsbeispiele für flüssige Wirkstoffe der Formel I (% = Gewichtsprozent)

| 2.1 Emulsions-Konzentrate | a) | b) | c) |
|---|---|---|---|
| Wirkstoff aus den Tabellen 1-3 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylenglykol-ether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2 Lösungen | a) | b) | c) | d) |
|---|---|---|---|---|
| Wirkstoff aus den Tabellen 1-3 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

(MG = Molekulargewicht)

Die Lösungen sind zur Anwendung in Form kleinster Tropfen geeignet.

| 2.3 Granulate | a) | b) |
|---|---|---|
| Wirkstoff aus den Tabellen 1-3 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) |
|---|---|---|
| Wirkstoff aus den Tabellen 1-3 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| 2.5 Spritzpulver | a) | b) | c) |
|---|---|---|---|
| Wirkstoff aus den Tabellen 1-3 | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2.6 Emulsions-Konzentrat

Wirkstoff aus den Tabellen 1-3 10 %

Octylphenolpolyethylenglykolether 3 %
(4-5 Mol Ethylenoxid)
Ca-Dodecylbenzolsulfonat 3 %
Ricinusölpolyglykolether (35 Mol Ethylenoxid) 4 %
Cyclohexanon 30 %
Xylolgemisch 50 %

Aus diesem Konzentrat können durch Verdünnen mit Wasser Emulsionen jeder gewünschten Konzentration hergestellt werden.

| 2.7 Stäubemittel | a) | b) |
|---|---|---|
| Wirkstoff aus den Tabellen 1-3 | 5 % | 8 % |
| Talkum | 95 % | - |
| Kaolin | - | 92 % |

Man erhält anwendungsfertige Stäubemittel, indem der Wirkstoff mit den Trägern vermischt und auf einer geeigneten Mühle vermahlen wird.

2.8 Extruder Granulat

Wirkstoff aus den Tabellen 1-3 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert und anschliessend im Luftstrom getrocknet.

2.9 Umhüllungs-Granulat

Wirkstoff aus den Tabellen 1-3 3 %
Polyethylenglykol (MG 200) 3 %
Kaolin 94 %
(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.10 Suspensions-Konzentrat

Wirkstoff aus den Tabellen 1-3 40 %
Ethylenglykol 10 %
Nonylphenolpolyethylenglykolether 6 %
(15 Mol Ethylenoxid)
N-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff wird mit den Zusatzstoffen innig vermischt. Man erhält so ein Suspensions-Konzentrat. aus welchem durch Verdünnen mit Wasser Suspensionen jeder gewünschten Konzentration hergestellt werden können.

Biologische Beispiele

3.1 Wirkung gegen Meloidogyne incognita

Eier von Meloidogyne incognita werden in Sand eingemischt. Dieses Gemisch wird dann in 200 ml fassende Tontöpfe eingebracht (5000 Eier pro Topf). Am gleichen Tag wird pro Topf eine 3 Wochen alte Tomatenpflanze gepflanzt und der formulierte Wirkstoff mittels Drenchapplikation in die Töpfe hineingegeben (0,0006 % Aktivsubstanz bezogen auf das Bodenvolumen). Die eingetopften Pflanzen werden nun bei einer Temperatur von $26 \pm 1°C$ und einer relativen Luftfeuchtigkeit von 60 % in einem Gewächshaus aufgestellt. Nach Ablauf von 4 Wochen erfolgt eine Evaluierung durch Untersuchung der Pflanzen auf Wurzelgallbildung nach dem sogenannten Wurzelgallen-Index ("Root-Knot Index").

Verbindungen aus den Tabellen 1-3 zeigen gegen Meloidogyne incognita durch weitgehende Reduktion der Wurzelgallbildung gute Wirksamkeit. Unbehandelte jedoch infizierte Kontrollpflanzen weisen dagegen starke Wurzelgallbildung auf (= 100 %). So hemmen z.B. die Verbindungen Nr. 1.1, 1.2, 1.3. 1.4 und 1.18 im obigen Versuch die Wurzelgallbildung fast vollständig (0-10 % Restbefall).

## Patentansprüche

1. Verbindungen der Formel I

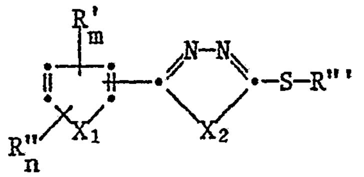

(I),

in welcher
R′ $C_1$-$C_5$-Alkyl, Halogen, Trifluormethyl oder $C_1$-$C_3$-Alkoxy;
R″ $C_1$-$C_5$-Alkyl oder Halogen; und
R‴ unsubstituiertes oder durch 1 bis 6 Halogenatome substituiertes $C_1$-$C_5$-Alkyl, unsubstituiertes oder durch 1 bis 6 Halogenatome substituiertes $C_1$-$C_5$-Alkenyl oder unsubstituiertes oder durch 1 bis 6 Halogenatome substituiertes $C_1$-$C_5$-Alkinyl bedeuten;
m für 0, 1, 2 oder 3 und
n für 0 oder 1 stehen; und
$X_1$ und $X_2$ unabängig voneinander Sauerstoff oder Schwefel darstellen.

2. Verbindungen der Formel I gemäss Anspruch 1, in denen
R′ $C_1$-$C_5$-Alkyl, Halogen, Trifluormethyl oder $C_1$-$C_3$-Alkoxy;
R″ $C_1$-$C_5$-Alkyl oder Halogen; und
R‴unsubstituiertes oder durch 1 bis 6 Halogenatome substituiertes $C_1$-$C_5$-Alkyl, unsubstituiertes oder durch 1 bis 6 Halogenatome substituiertes $C_1$-$C_5$-Alkenyl oder unsubstituiertes oder durch 1 bis 6 Halogenatome substituiertes $C_1$-$C_5$-Alkinyl bedeuten;
m für 0, 1, 2 oder 3 und
n für 0 oder 1 stehen; und
$X_1$ und $X_2$ unabhängig voneinander Sauerstoff oder Schwefel darstellen, mit der Massgabe, dass wenn m und n zusammen Null sind oder wenn $X_1$ Schwefel und R′ und R″ zusammen 3 Chloratome darstellen, R‴ nicht Alkenyl oder Alkinyl sein darf.

3. Verbindungen der Formel I gemäss Anspruch 1, in denen
R′ $C_1$-$C_2$-Alkyl, Halogen, Trifluormethyl oder $C_1$-$C_2$-Alkoxy;
R″ $C_1$-$C_2$-Alkyl oder Halogen;
R‴ unsubstituiertes oder durch 1 bis 3 Halogenatome substituiertes $C_1$-$C_3$-Alkyl, unsubstituiertes oder durch 1 bis 3 Halogenatome substituiertes $C_1$-$C_3$-Alkenyl oder unsubstituiertes oder durch 1 bis 3 Halogenatome substituiertes $C_1$-$C_3$-Alkinyl bedeuten;
m für 0, 1 oder 2 und
n für 0 oder 1 stehen; und
$X_1$ und $X_2$ unabhängig voneinander Sauerstoff oder Schwefel darstellen.

4. Verbindungen der Formel I gemäss Anspruch 1, in denen R′ $C_1$-$C_2$-Alkyl, Halogen, Trifluormethyl oder $C_1$-$C_2$-Alkoxy;
R″ $C_1$-$C_2$-Alkyl oder Halogen;
R‴ unsubstituiertes oder durch 1 bis 3 Halogenatome substituiertes $C_1$-$C_3$-Alkyl, unsubstituiertes oder durch 1 bis 3 Halogenatome substituiertes $C_1$-$C_3$-Alkenyl oder unsubstituiertes oder durch 1 bis 3 Halogenatome substituiertes $C_1$-$C_3$-Alkinyl bedeuten;
m für 0, 1 oder 2 und
n für 0 oder 1 stehen; und
$X_1$ und $X_2$ unabhängig voneinander Sauerstoff oder Schwefel darstellen, mit der Massgabe, dass wenn m und n zusammen Null sind oder wenn $X_1$ Schwefel und R′ und R″ zusammen 3 Chloratome darstellen, R‴ nicht Alkenyl oder Alkinyl sein darf.

5. Verbindungen der Formel I gemäss Anspruch 1, in denen
R′ Methyl, Chlor, Brom oder Methoxy;
R″ Methyl, Chlor Brom oder Jod;
R‴ unsubstituiertes oder durch 1 bis 3 Fluoratome substituiertes $C_2$-$C_3$-Alkyl, unsubstituiertes oder durch 1 bis 3 Fluoratome substituiertes $C_1$-$C_3$-Alkenyl oder unsubstituiertes oder durch 1 bis 3 Fluoratome substituiertes $C_1$-$C_3$-Alkinyl bedeuten; m und n unabhängig für oder 1 stehen; und
$X_1$ und $X_2$ unabhängig voneinander Sauerstoff oder Schwefel darstellen.

6. Verbindungen der Formel I gemäss Anspruch 1, in denen
R′ Methyl, Chlor, Brom oder Methoxy;
R″ Methyl, Chlor Brom oder Jod;
R‴ unsubstituiertes oder durch 1 bis 3 Fluoratome substituiertes $C_1$-$C_3$-Alkyl, unsubstituiertes oder

durch 1 bis 3 Fluoratome substituiertes $C_1$-$C_3$-Alkenyl oder unsubstituiertes oder durch 1 bis 3 Fluoratome substituiertes $C_1$-$C_3$-Alkinyl bedeuten;
m und n unabhängig für 0 oder 1 stehen; und
$X_1$ und $X_2$ unabhängig voneinander Sauerstoff oder Schwefel darstellen, mit der Massgabe, dass wenn m und n zusammen Null sind oder wenn $X_1$ Schwefel und R′ und R″ zusammen 3 Chloratome darstellen, R‴ nicht Alkenyl oder Alkinyl sein darf.

7. Verbindungen der Formel I gemäss Anspruch 1, in denen
R′ Methyl, Chlor, Brom oder Methoxy;
R″ Methyl, Chlor oder Brom; und
R‴ unsubstituiertes oder durch 1 bis 3 Fluoratome substituiertes $C_1$-$C_3$-Alkyl bedeuten;
m und n unabhängig voneinander für 0 oder 1 stehen; und $X_1$ und $X_2$ unabhdängig voneinander Sauerstoff oder Schwefel darstellen.

8. Verbindungen der Formel I gemäss Anspruch 1, in denen
R′ Methyl, Chlor, Brom oder Methoxy;
R″ Methyl, Chlor oder Brom; und
R‴ $CHF_2$ bedeuten; und
m und n unabhängig voneinander für 0 oder 1 stehen; und
$X_1$ und $X_2$ unabhängig voneinander Sauerstoff oder Schwefel darstellen.

9.Eine Verbindung der Formel I nach Anspruch 1, ausgewählt aus der Gruppe
2-Difluormethylthio-5-thien-2-yl-1,3,4-thiadiazol,
2-Difluormethylthio-5-(5-chlorthien-2-yl)-1,3,4-thiadiazol,
2-Difluormethylthio-5-(5-methylthien-2-yl)1,3,4-thiadiazol
2-Difluormethylthio-5-(5-bromthien-2-yl)-1,3,4-thiadiazol,
2-Difluormethylthio-5-thien-2-yl-1,3,4-oxadiazol,
2-Difluormethylthio-5-(chlorthien-2-yl)-1,3,4-oxadiazol,
2-Difluormethylthio-5-(5-methylthien-2-yl)-1,3,4-oxadiazol,
2-Difluormethylthio-5-(furan-2-yl)-1,3,4-thiadiazol,
2-Difluormethylthio-5-(2-methylfuran-3-yl)-1,3,4-thiadiazol,
2-Difluormethylthio-5-(4-methylthien-2-yl)-1,3,4-thiadiazol,
2-Difluormethylthio-5-(furan-2-yl)-1,3,4-oxadiazol

10. Verfahren zur Herstellung der Verbindungen der Formel I gemäss Anspruch 1, dadurch gekennzeichnet, dass man
a) in einer Kondensationsreaktion eine Verbindung der Formel IIa

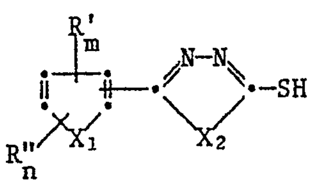

(IIa)

oder eine Verbindung der Formel IIb

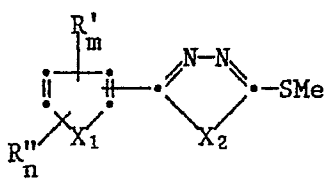

(IIb)

mit einer Verbindung der Formel IIIa
Hal-R‴ (IIIa)
in einem inerten Lösungsmittel oder Lösungsmittel-Gemisch bei erhöhter Temperatur gegebenenfalls in Gegenwart eines Katalysators und gegebenenfalls bei erhöhtem Druck umsetzt, wobei die Umsetzung einer Verbindung der Formel IIa in Gegenwart einer Base verläuft, oder
b) in einer Anlagerungsreaktion eine Verbindung der Formel IIa

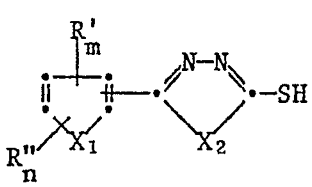

(IIa)

mit einer Verbindung der Formel IIIb

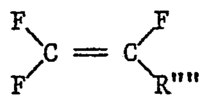

(IIIb)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ia

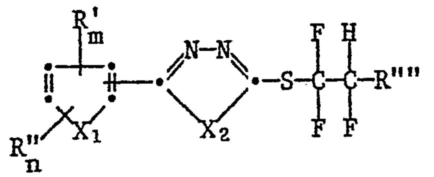

(Ia)

oder zu einer Verbindung der Formel Ib

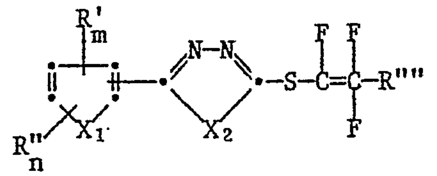

(Ib)

führt, wobei in den vorstehend genannten Formeln IIa, IIb, Ia, Ib, IIIa und IIIb Me für ein Alkalimetall oder für Ammonium steht, Hal Halogen, vorzugsweise Chlor, Brom oder Jod bedeutet, und R'''' Fluor oder Trifluormethyl darstellt, während R', R'', R''', m, n, $X_1$ und $X_2$ die unter Formel I angegebenen Bedeutungen besitzen.

11. Schädlingsbekämpfungsmittel zur Bekämpfung oder Verhütung des Befalls von Pflanzen durch Nematoden, dadurch gekennzeichnet, dass es als aktive Komponente mindestens eine Verbindung der Formel I gemäss Anspruch 1 enthält.

12. Mittel gemäss Anspruch 11, dadurch gekennzeichnet, dass es als aktive Komponente mindestens eine Verbindung der Formel I gemäss der Ansprüche 2 bis 8 enthält.

13. Mittel zur Bekämpfung von Nematoden gemäss Anspruch 11, dadurch gekennzeichnet, dass es als aktive Komponente mindestens eine Verbindung der Formel I gemäss Anspruch 9 enthält.

14. Mittel zur Bekämpfung von Nematoden gemäss Anspruch 11, dadurch gekennzeichnet, dass es als aktive Komponente mindestens eine Verbindung der Formel I gemäss Anspruch 10 enthält.

15. Mittel nach einem der Ansprüche 11 bis 14, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

16. Mittel nach Anspruch 15, dadurch gekennzeichnet, dass es 0,1 bis 95 % eines Wirkstoffes der Formel I, 99,8 bis 5 % eines festen oder flüssigen Zusatzstoffes und 0,1 bis 25 % eines Tensides enthält.

17. Verfahren zur Herstellung eines agrochemischen Mittels, dadurch gekennzeichnet, dass mindestens eine Verbindung der Formel I gemäss einem der Ansprüche 11 bis 15 mit geeigneten festen oder flüssigen Zusatzstoffen und Tensiden innig vermischt.

18. Verfahren zur Bekämpfung oder Verhütung eines Befalls von Kulturpflanzen durch Nematoden, dadurch gekennzeichnet, dass man eine Verbindung der Formel I gemäss einem der Ansprüche 1 bis 9 auf die Pflanze oder deren Standort appliziert.

19. Verwendung von Verbindungen der Formel I gemäss Anspruch 1 zur Bekämpfung und/oder zur präventiven Verhütung eines Befalls von Pflanzen durch Nematoden.

20. Verwendung gemäss Anspruch 19, von Verbindungen der Formel I gemäss einem der Ansprüche 2 bis 9.

21. Verwendung gemäss Anspruch 19, dadurch gekennzeichnet, dass es sich bei den Nematoden um pflanzenparasitäre Arten handelt.

22. Verwendung gemäss Anspruch 21 gegen Nematoden der Gattung Meloidogyne, Heterodera oder Globodera.

Patentansprüche für die folgenden Vertragsstaaten: (AT und ES)

1. Schädlingsbekämpfungsmittel zur Bekämpfung oder Verhütung des Befalls von Pflanzen durch Nematoden, dadurch gekennzeichnet, dass es als aktive Komponente mindestens eine Verbindung der Formel I

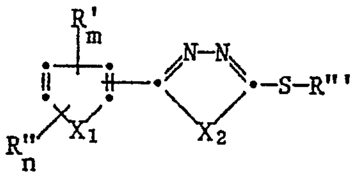 (I),

in welcher
R′ $C_1$-$C_5$-Alkyl, Halogen, Trifluormethyl oder $C_1$-$C_3$-Alkoxy;
R″ $C_1$-$C_5$-Alkyl oder Halogen; und
R‴ unsubstituiertes oder durch 1 bis 6 Halogenatome substituiertes $C_1$-$C_5$-Alkyl, unsubstituiertes oder durch 1 bis 6 Halogenatome substituiertes $C_1$-$C_5$-Alkenyl oder unsubstituiertes oder durch 1 bis 6 Halogenatome substituiertes $C_1$-$C_5$-Alkinyl bedeuten;
m für 0, 1, 2 oder 3 und
n für 0 oder 1 stehen; und
$X_1$ und $X_2$ unabhängig voneinander Sauerstoff oder Schwefel darstellen, enthält.

2. Mittel gemäss Anspruch 1, enthaltend Verbindungen in denen
R′ $C_1$-$C_5$-Alkyl, Halogen, Trifluormethyl oder $C_1$-$C_3$-Alkoxy;
R″ $C_1$-$C_5$-Alkyl oder Halogen; und
R‴unsubstituiertes oder durch 1 bis 6 Halogenatome substituiertes $C_1$-$C_5$-Alkyl, unsubstituiertes oder durch 1 bis 6 Halogenatome substituiertes $C_1$-$C_5$-Alkenyl oder unsubstituiertes oder durch 1 bis 6 Halogenatome substituiertes $C_1$-$C_5$-Alkinyl bedeuten;
m für 0, 1, 2 oder 3 und
n für 0 oder 1 stehen; und
$X_1$ and $X_2$ unabhängig voneinander Sauerstoff oder Schwefel darstellen, mit der Massgabe, dass wenn m und n zusammen Null sind oder wenn $X_1$ Schwefel und R′ und R″ zusammen 3 Chloratome darstellen, R‴ nicht Alkenyl oder Alkinyl sein darf.

3. Mittel gemäss Anspruch 1, enthaltend Verbindungen in denen
R′ $C_1$-$C_2$-Alkyl, Halogen, Trifluormethyl oder $C_1$-$C_2$-Alkoxy;
R″ $C_1$-$C_2$-Alkyl oder Halogen;
R‴ unsubstituiertes oder durch 1 bis 3 Halogenatome substituiertes $C_1$-$C_3$-Alkyl, unsubstituiertes oder durch 1 bis 3 Halogenatome substituiertes $C_1$-$C_3$-Alkenyl oder unsubstituiertes oder durch 1 bis 3 Halogenatome substituiertes $C_1$-$C_3$-Alkinyl bedeuten;
m für 0, 1 oder 2 und
n für 0 oder 1 stehen; und
$X_1$ und $X_2$ unabhängig voneinander Sauerstoff oder Schwefel darstellen.

4. Mittel gemäss Anspruch 1, enthaltend Verbindungen in denen
R′ $C_1$-$C_2$-Alkyl, Halogen, Trifluormethyl oder $C_1$-$C_2$-Alkoxy;
R″ $C_1$-$C_2$-Alkyl oder Halogen;
R‴ unsubstituiertes oder durch 1 bis 3 Halogenatome substituiertes $C_1$-$C_3$-Alkyl, unsubstituiertes oder durch 1 bis 3 Halogenatome substituiertes $C_1$-$C_3$-Alkenyl oder unsubstituiertes oder durch 1 bis 3 Halogenatome substituiertes $C_1$-$C_3$-Alkinyl bedeuten;
m für 0, 1 oder 2 und
n für 0 oder 1 stehen; und
$X_1$ und $X_2$ unabhängig voneinander Sauerstoff oder Schwefel darstellen, mit der Massgabe, dass wenn m und n zusammen Null sind oder wenn $X_1$ Schwefel und R′ und R″ zusammen 3 Chloratome darstellen, R‴ nicht Alkenyl oder Alkinyl sein darf.

5. Mittel gemäss Anspruch 1, enthaltend Verbindungen in denen
R′ Methyl, Chlor, Brom oder Methoxy;
R″ Methyl, Chlor Brom oder Jod;
R‴ unsubstituiertes oder durch 1 bis 3 Fluoratome substituiertes $C_1$-$C_3$-Alkyl, unsubstituiertes oder durch 1 bis 3 Fluoratome substituiertes $C_1$-$C_3$-Alkenyl oder unsubstituiertes oder durch 1 bis 3 Fluoratome substituiertes $C_1$-$C_3$-Alkinyl bedeuten;
m und n unabhängig für 0 oder 1 stehen; und
$X_1$ und $X_2$ unabhängig voneinander Sauerstoff oder Schwefel darstellen.

6. Mittel gemäss Anspruch 1, enthaltend Verbindungen in denen
R′ Methyl, Chlor, Brom oder Methoxy;
R″ Methyl, Chlor Brom oder Jod:
R‴ unsubstituiertes oder durch 1 bis 3 Fluoratome substituiertes $C_1$-$C_3$-Alkyl, unsubstituiertes oder durch 1 bis 3 Fluoratome substituiertes $C_1$-$C_3$-Alkenyl oder unsubstituiertes oder durch 1 bis 3 Fluoratome substituiertes $C_1$-$C_3$-Alkinyl bedeuten;
m und n unabhängig für 0 oder 1 stehen; und
$X_1$ und $X_2$ unabhängig voneinander Sauerstoff oder Schwefel darstellen, mit der Massgabe, dass wenn m und n zusammen Null sind oder wenn $X_1$ Schwefel und R′ und R″ zusammen 3 Chloratome darstellen, R‴

nicht Alkenyl oder Alkinyl sein darf.

7. Mittel gemäss Anspruch 1, enthaltend Verbindungen in denen R′ Methyl, Chlor, Brom oder Methoxy; R″ Methyl, Chlor oder Brom; und R‴ unsubstituiertes oder durch 1 bis 3 Fluoratome substituiertes $C_1$-$C_3$-Alkyl bedeuten; m und n unabhängig voneinander für 0 oder 1 stehen; und $X_1$ und $X_2$ unabhdängig voneinander Sauerstoff oder Schwefel darstellen.

8. Mittel gemäss Anspruch 1, enthaltend Verbindungen in denen
R′ Methyl, Chlor, Brom oder Methoxy;
R″ Methyl, Chlor oder Brom; und
R‴ $CHF_2$ bedeuten; und
m und n unabhängig voneinander für 0 oder 1 stehen; und
$X_1$ und $X_2$ unabhängig voneinander Sauerstoff oder Schwefel darstellen.

9. Mittel nach Anspruch 1, enthaltend eine Verbindung ausgewählt aus der Gruppe
2-Difluormethylthio-5-thien-2-yl-1,3,4-thiadiazol,
2-Difluormethylthio-5-(5-chlorthien-2-yl)-1,3,4-thiadiazol,
2-Difluormethylthio-5-(5-methylthien-2-yl)-1,3,4-thiadiazol,
2-Difluormethylthio-5-(5-bromthien-2-yl)-1,3,4-thiadiazol,
2-Difluormethylthio-5-thien-2-yl-1,3,4-oxadiazol,
2-Difluormethylthio-5-(chlorthien-2-yl)-1,3,4-oxadiazol,
2-Difluormethylthio-5-(5-methylthien-2-yl)-1,3,4-oxadiazol,
2-Difluormethylthio-5-(furan-2-yl)-1,3,4-thiadiazol,
2-Difluormethylthio-5-(2-methylfuran-3-yl)-1,3,4-thiadiazol,
2-Difluormethylthio-5-(4-methylthien-2-yl)-1,3,4-thiadiazol,
2-Difluormethylthio-5-(furan-2-yl)-1,3,4-oxadiazol.

10. Verfahren zur Herstellung der Verbindungen der Formel I gemäass Anspruch 1, dadurch gekennzeichnet, dass man
a) in einer Kondensationsreaktion eine Verbindung der Formel IIa

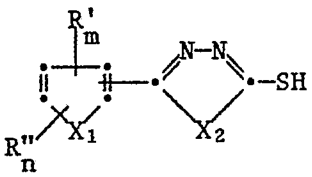
(IIa)

oder eine Verbindung der Formel IIb

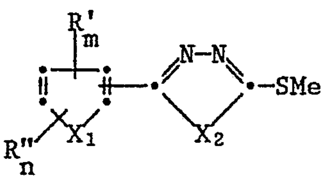
(IIb)

mit einer Verbindung der Formel IIIa
Hal-R‴ (IIIa)
in einem inerten Lösungsmittel oder Lösungsmittel-Gemisch bei erhöhter Temperatur gegebenenfalls in Gegenwart eines Katalysators und gegebenenfalls bei erhöhtem Druck umsetzt, wobei die Umsetzung einer Verbindung der Formel IIa in Gegenwart einer Base verläuft, oder
b) in einer Anlagerungsreaktion eine Verbindung der Formel IIa

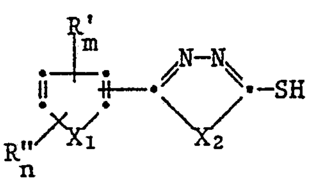
(IIa)

mit einer Verbindung der Formel IIIb

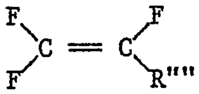 (IIIb)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ia

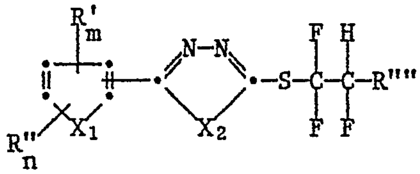 (Ia)

oder zu einer Verbindung der Formel Ib

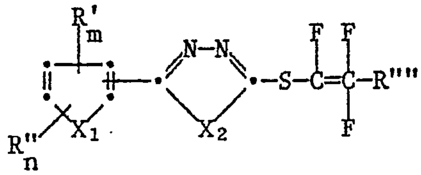 (Ib)

führt, wobei in den vorstehend genannten Formeln IIa, IIb, Ia, Ib, IIIa und IIIb Me für ein Alkalimetall oder für Ammonium steht, Hal Halogen, vorzugsweise Chlor, Brom oder Jod bedeutet, und R'''' Fluor oder Trifluormethyl darstellt, während R', R'', R''', m, n, $X_1$ und $X_2$ die unter Formel I angegebenen Bedeutungen besitzen.

11. Verfahren zur Herstellung eines agrochemischen Mittels, dadurch gekennzeichnet, dass mindestens eine Verbindung der Formel I gemäss einem der Ansprüche 1 bis 9 mit geeigneten festen oder flüssigen Zusatzstoffen und Tensiden innig vermischt.

12. Verfahren zur Bekämpfung oder Verhütung eines Befalls von Kulturpflanzen durch Nematoden, dadurch gekennzeichnet, dass man eine Verbindung der Formel I gemäss einem der Ansprüche 1 bis 9 auf die Pflanze oder deren Standort appliziert.

13. Verwendung von Verbindungen der Formel I gemäss Anspruch 1 zur Bekämpfung und/oder zur präventiven Verhütung eines Befalls von Pflanzen durch Nematoden.

14. Verwendung gemäss Anspruch 13, von Verbindungen der Formel I gemäss einem der Ansprüche 1 bis 9.

15. Verwendung gemäss Anspruch 13, dadurch gekennzeichnet, dass es sich bei den Nematoden um pflanzenparasitäre Arten handelt.

16. Verwendung gemäss Anspruch 15 gegen Nematoden der Gattung Meloidogyne, Heterodera oder Globodera.